# EUROPÄISCHE PATENTSCHRIFT

(11) **EP 1 186 312 B1**
(45) Veröffentlichungstag und Bekanntmachung des Hinweises auf die Patenterteilung: **28.12.2005**
(21) Anmeldenummer: 01114759.2
(22) Anmeldetag: 25.06.2001
(51) Int. Cl.: A61M 5/24

(54) **Austragsvorrichtung für Medien**
Dispensing device for substances
Dispositif distributeur de substances

(30) Priorität: 27.07.2000 DE 10036594
(43) Veröffentlichungstag der Anmeldung: 13.03.2002
(73) Patentinhaber: Ing. Erich Pfeiffer GmbH, 78315 Radolfzell (DE)
(72) Erfinder: Stadelhofer, Peter, 78224 Singen (DE)
(74) Vertreter: Patentanwälte Ruff, Wilhelm, Beier, Dauster & Partner

(56) Entgegenhaltungen:
- EP-A- 1 066 847
- WO-A-91/10460
- US-A- 4 962 868
- US-A- 5 427 280

## Beschreibung

Die Erfindung betrifft eine Austragvorrichtung für Medien, wie sie beispielsweise aus der EP 0 245 895 A1 bekannt ist. Eine derartige Austragvorrichtung für Medien ist insbesondere für den Austrag von wenigstens einem pharmazeutischen Wirkstoff enthaltenden Medien bestimmt.

Bei derartigen Austragvorrichtungen ist das auszutragende Medium in einem Behälter bevorratet. Dieser Behälter weist voneinander getrennte Kammern auf. In jede der Kammer ist jeweils eine Medienkomponente eingebracht, wobei die Medienkomponenten zusammen das auszutragende Medium ergeben. Der Behälter wiederum ist in einem Gehäuse angeordnet, das eine Austragöffnung zum Austrag von Medium aufweist. Dabei ist ein bzgl. dem Gehäuse relativ bewegliches Betätigungsmittel vorgesehen. Durch die Betätigung des Betätigungsmittels wird zunächst eine Verbindung zwischen den Kammern des Behälters hergestellt.

Bei Austragvorrichtungen von Medien sind auszutragende Medien in einem Behälter bevorratet. Dabei gibt es Medien, die in ihrer anwendungsbereiten Darreichungsform nicht dauerhaft stabil und lagerfähig sind. Damit es möglich ist, derartige Medien über einen längeren Zeitraum hinweg, der insbesondere einen angemessenen Zeitraum für die Zwischenlagerung, für die Verteilung des hergestellten Mediums über den Hersteller zum Großhändler und anschließend zum Einzelhändler, beispielsweise Apotheken, und eine hinreichende Aufbrauchfrist des Mediums sicherzustellen, wurde vorgeschlagen, das Medium in Form von einzeln, jeweils stabilen oder stabileren Medienkomponenten gesondert zu bevorraten und das Vermischen der Medienkomponenten erst unmittelbar vor der Anwendung vorzunehmen. Da das Vermengen der Komponenten meist einer genauen Dosierung der Komponenten bedarf, kann dies nicht mehr ohne weiteres vom Laien selbständig durchgeführt werden. Es bedarf oft zumindest einer geschulten Hilfskraft, um diese Tätigkeit vorzunehmen und dann anschließend das Medium beim Endanwender, dem Patienten, zu applizieren.

Beispiele für Medien, die in unterschiedliche Teilkomponenten aufgeteilt längerfristig erhaltbar sind und die als Mischung zusammen nicht dauerhaft stabil sind, sind vielfach bei pharmazeutischen Anwendungen zu finden. Insbesondere wenn Teil des Mediums ein gefriergetrocknetes Pulver (ein sog. Lyophilisat) ist, das in einer wässrigen Trägerflüssigkeit aufzulösen ist oder mit dieser zusammen ausgetragen werden soll, ist das Stoffgemenge nicht stabil. Daneben gibt es aber auch eine Reihe von Anwendungen, bei denen zwei flüssige Medienkomponenten in ihrer Mischung nicht stabil sind, jedoch einzeln und getrennt voneinander durchaus langzeitlagerfähig sind.

Ein Spender, bei dem Medienkomponenten getrennt voneinander in einem Behälter gelagert und gemeinsam vermengt austragbar sind, ist beispielsweise aus der gattungsgemäß zugrunde gelegten EP 0 245 895 A1 bekannt. Bei einem derartigen Spender ist ein Betätigungsmittel vorgesehen, um den Vermischungsvorgang der Medienkomponenten miteinander durchzuführen und anschließend das Medium auszutragen. Dabei müssen zum Vermischen der Medienkomponenten miteinander mehrere Pumphübe mit dem Betätigungsmittel durchgeführt werden. Nach Abschluß der Pumphübe, bei vollständiger Vermengung der Medienkomponenten miteinander, erfolgt ein Austraghub, wobei während des Austraghubes die Gesamtmenge des Mediums ausgetragen wird.

Dabei ist es umständlich, daß zum Vermengen der Medienkomponenten eine Vielzahl von Pumphüben durchgeführt werden muß.

Andererseits hat es sich erwiesen, daß es günstig sein kann, ein Medium in mehreren, definierten Teilchargen auszutragen. Dies ist beispielsweise dann der Fall, wenn das Medium nasal appliziert werden soll und eine möglichst gleiche Menge Medium in die beiden Nasenlöcher eingebracht werden soll. Es hat sich erwiesen, daß beispielsweise bei der Applikation von Migränemitteln eine nasale Applikation von Vorteil ist, da dadurch eine rasche Wirkstoffabsorption durch den Körper stattfindet. Gleichzeitig können dadurch auch Wirkstoffe appliziert werden, die ansonsten unter Umständen nicht in Tablettenform verabreicht werden können, sondern in Form von Spritzen appliziert werden müßten und daher nur von einer geschulten Kraft verabreicht werden können. Dabei ist die Anwendung einer derartigen Medikamentenapplikation nicht allein auf Migränemittel beschränkt. Es können auch andere Formen von Schmerzmitteln sowie weitere pharmazeutische Wirkstoffe derart nasal appliziert werden. Es kommt dabei allein auf die Fähigkeit an, den durch die Nase applizierten Wirkstoff durch den Körper aufzunehmen.

Dabei ist es nicht bekannt, dass vor Durchführung der Teilausträge des Mediums ein Mischvorgang von Medienkomponenten stattfinden muss, um das auszutragende Medium zu erzeugen.

Die WO 91/10460 beschreibt eine Injektionsspritze mit einer Mischvorrichtung gemäß dem Oberbegriff des Anspruchs 1. Die Injektionsnadel durchdringt beim Einsetzen des Spritzenzylinders in die Vorrichtung einen Stopfen, der den Spritzenzylinder begrenzt.

Auch die EP 1 066 846 A1 betrifft eine Injektionsspritze, bei der der Endverschluss von der Rückseite der Injektionsnadel durchdrungen wird.

Die US 5 427 280 beschreibt einen Zerstäuber für zwei Teilhübe, die mittels einer Kulissenführung voneinander abgegrenzt sind. Hier wird keine Komponentenmischung vorgenommen.

Schließlich beschreibt die US 4 962 868 einen Spender, der nach Art einer Injektionsspritze arbeitet und bei dem im Gehäuse eine Kulisse mit federnden Rasten vorgesehen ist, die aufeinander folgende Teilhübe voneinander abgrenzen sollen.

Diese Aufgabe wird durch den Anspruch 1 gelöst.

Die Austragvorrichtung, die insbesondere zum Austragen eines wenigstens einen pharmazeutischen Wirkstoff enthaltenden Mediums bestimmt ist, weist einen Behälter auf, in dem das Medium in Form von Medienkomponenten bevorratet ist, wobei die Medienkomponenten in voneinander getrennten Kammern eingebracht sind. Die Medienkomponenten ergeben zusammen das auszutragende Medium. Der Behälter ist in einem Gehäuse angeordnet, das eine Austragöffnung zum Austrag von Medium aufweist. Dabei ist ein Betätigungsmittel vorgesehen, das bzgl. dem Gehäuse relativ beweglich ist. Darunter ist auch eine Anordnung zu verstehen, bei der der Behälter mittelbar in einer Hülse angeordnet ist, die im Gehäuse gehalten ist, wobei das Betätigungsmittel relativ beweglich zu der Hülse angeordnet ist. Eine Betätigung des Betätigungsmittels führt dabei zunächst zur Herstellung einer Verbindung zwischen den Kammern des Behälters. Der Betätigungsweg des Betätigungsmittels ist gemäß der Erfindung in einen ersten Teilbetätigungsweg und in eine Anzahl nachfolgender Teilbetätigungswege unterteilt. Während des ersten Betätigungsweges findet dabei das Vermischen der Medienkomponenten zu dem auszutragenden Medium statt. Jeder der nachfolgenden Teilbetätigungen des Betätigungsmittels ist der zerstäubte Austrag einer definierten Teilcharge des Mediums zugeordnet. Die Anzahl der nachfolgenden Teilbetätigungen beträgt vorteilhafterweise wenigstens zwei, insbesondere zwei. Es ist aber auch möglich, daß lediglich eine nachfolgende Teilbetätigung vorgesehen ist, also das gesamte auszutragende Medium in einer nachfolgenden Teilbetätigung auszutragen. Insbesondere hierbei ist es sinnvoll, wenn die erste Teilbetätigung und die erste nachfolgende Teilbetätigung mittels einer ununterbrochenen, durchgehenden Betätigung des Betätigungsmittels erfolgt. Besonders vorteilhaft ist es bei einer derartigen Ausbildung der Austragvorrichtung, daß nicht eine Vielzahl von Teilbetätigungen des Betätigungsmittels erforderlich ist, um die Vermischung der Medienkomponenten zum Medium zu erzeugen, sondern dieses durch eine einzige Teilbetätigung erfolgt. Die weiteren Betätigungen dienen dabei jeweils zum Austrag einer definierten Teilcharge.

Gemäß vorteilhafter Ausgestaltung der Erfindung sind die Kammern des Behälters durch Stopfen voneinander getrennt, die im Behälter angeordnet sind. Darüber hinaus ist es vorteilhaft, wenn an dem Behälter Überströmkanäle ausgebildet sind, die derart positioniert sind, daß während der ersten Teilbetätigung des Betätigungsmittels die die Kammern voneinander trennenden Stopfen in eine Position im Bereich der Überströmkanäle verbringbar sind, so daß über die Überströmkanäle die Kammern miteinander verbunden sind.

Gemäß bevorzugter Ausbildung der Erfindung ist zur Unterteilung des Betätigungsweges in die Teilbetätigungswege eine Kulissenführung vorgesehen. Bevorzugt weist die Kulissenführung Druckpunktmittel auf, wobei zu Beginn jeder nachfolgenden Teilbetätigung Druckpunktmittel zu überwinden sind. Vorteilhaft ist es, wenn die Kulissenführung für jede Teilbetätigung einen linearen, geradlinigen Führungsabschnitt aufweist, wobei die Führungsabschnitte derart zueinander versetzt sind, daß sie jeweils durch einen Anschlag begrenzt sind und daß zwischen zwei Teilbetätigungen jeweils eine von der Teilbetätigung verschiedene Umschaltbetätigung durchzu führen ist. Bevorzugt werden dabei Ausgestaltungen, wobei wenigstens die Umschaltbetätigung zwischen zwei nachfolgenden Teilbetätigungen selbsttätig durchgeführt werden. Besonders bevorzugt werden dabei Ausgestaltungen, wobei zur selbsttätigen Durchführung der Umschaltbetätigung Kraftspeicher vorgesehen sind, die während der Durchführung der vorhergehenden Teilbetätigung vorgespannt werden.

Gemäß bevorzugter Ausgestaltung der Erfindung ist der Behälter in einer Hülse lagefest angeordnet. Die Hülse wiederum ist zum Gehäuse relativ beweglich angeordnet und von einer Ausgangsstellung in eine Austragstellung verbringbar. In der Ausgangsstellung wird der Behälter von der Hülse in dem Gehäuse der Austragvorrichtung ausgerichtet gehalten. Der Behälter ist hermetisch verschlossen. Von dieser Ausgangsstellung kann die Hülse in die Austragstellung verbracht werden, wobei während des Verbringens in diese Stellung eine fluidische Verbindung zwischen der ersten, zur Austragöffnung der Austragvorrichtung hin ausgerichteten Kammer hergestellt wird. Dabei wird es bevorzugt, wenn das Verbringen der Hülse von der Ausgangsstellung in die Austragstellung während der ersten Teilbetätigung erfolgt. Zur Herstellung der fluidischen Verbindung zwischen der ersten Kammer und der Austragöffnung wird ein behälterseitiger Abschlußstopfen, der vorzugsweise mit einem Crimp-Verschluß am Behälter befestigt ist, von einer gehäuseseitig angeordneten, einen Austragkanal aufweisenden Nadel durchstochen.

Es ist vorteilhaft, wenn die Kulissenführung zwischen Hülse und Betätigungsmittel ausgebildet ist und vorzugsweise der wenigstens eine Gleitstein an dem Betätigungsmittel und die wenigstens eine Kulissenbahn der Kulissenführung an der Hülse ausgebildet sind.

Vorteilhaft ist es weiterhin, wenn die Umschaltbetätigung zwischen der ersten Teilbetätigung und der ersten nachfolgenden Teilbetätigung durch ein Verdrehen des Betätigungsmittels gegenüber dem Gehäuse, bzw. falls vorgesehen gegenüber einer im Gehäuse angeordneten Hülse erfolgt, wobei vorzugsweise der Drehwinkel durch Anschlagkanten der Kulissenführung vorgegeben ist und wobei weiter vorzugsweise mittels einer Verzahnung ein Verdrehen der Hülse gegenüber dem Gehäuse verhindert wird.

Weiter vorteilhaft ist es, wenn der Behälter eine erste, der Austragöffnung der Austragvorrichtung zugewandte Kammer und wenigstens eine weitere Kammer aufweist. Die Kammern sind vorzugsweise mit im Behälter axial verschiebbaren Stopfen voneinander getrennt. Die letzte der Kammern ist ebenfalls mit einem Stopfen verschlossen, auf diesen wirkt das Betätigungsmittel ein.

Gemäß einer vorteilhaften Ausgestaltung weist die erste, der Austragöffnung zugewandte Kammer ein Volumen mit einer Gasblase auf, wobei während der ersten Teilbetätigung durch Kompression der Gasblase ein Kraftschluß zwischen Betätigungsmittel und Behälter herstellbar ist, mittels dem der Behälter von der Ausgangsstellung, in der der Behälter hermetisch verschlossen ist, in die Austragstellung, in der eine fluidische Verbindung zwischen Austragöffnung und erster Kammer hergestellt ist, verbringbar ist. Bei einer solchen Ausgestaltung wird mit Herstellen der fluidischen Verbindung der Kraftschluß über die Gasblase unterbrochen, also in einfacher Weise ein Weg-gesteuerter Kraftschluß erzeugt. Alternativ oder ergänzend hierzu ist es auch möglich, daß der die erste Teilbetätigung begrenzende Anschlag derart ausgebildet ist, daß der Behälter in eine Austragstellung verbracht wird, in der die fluidische Verbindung zwischen erster Kammer und Austragöffnung hergestellt ist.

Bevorzugt werden Ausgestaltungen, in denen der Behälter zwei Kammern aufweist, wobei in der ersten Kammer vorzugsweise eine trockene, insbesondere lösliche Medienkomponente, wie ein Lyophilisat, eingebracht ist und wobei in der zweiten Kammer ein Trägermedium, vorzugsweise ein insbesondere flüssiges Lösungsmittel als Medienkomponente eingebracht ist. Dabei ist es weiter vorteilhaft, wenn zwei, insbesondere einen gleich großen Austrag bewirkende nachfolgende Teilbetätigungen durchführbar sind.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird.

Ein Ausführungsbeispiel der Erfindung ist in der Zeichnung dargestellt und wird im folgenden näher erläutert; dabei zeigt:
- Fig. 1: dieSchnittdarstellung einer bevorzugten, unbetätigten Austragvorrichtung;
- Fig. 2: eine teilgeschnittene Seitenansicht einer bevorzugten, unbetätigten Austragvorrichtung, und
- Fig. 3: die teilgeschnittene Darstellung der Austragvorrichtung von Fig. 2 von der gegenüberliegenden Seite.

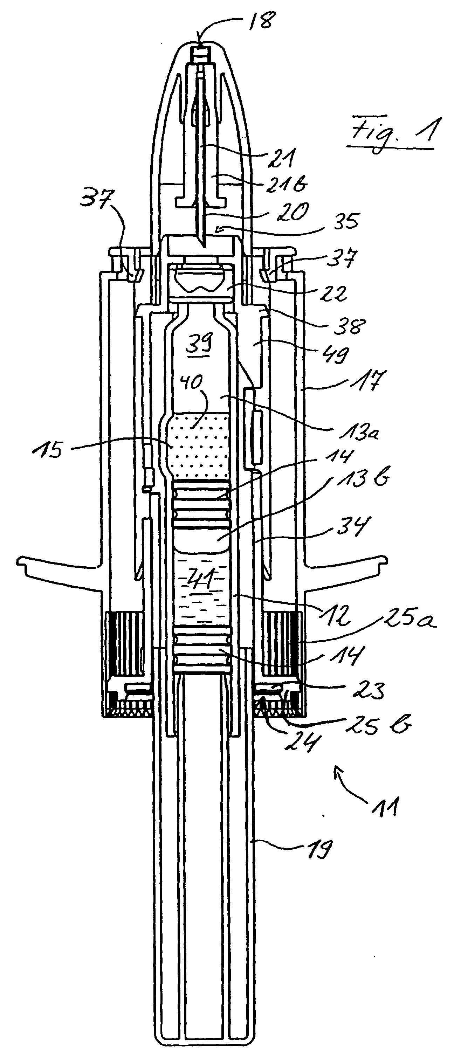

Die Fign. 1 bis 3 zeigen jeweils Darstellungen eines bevorzugten Spenders in seiner unbetätigten Ausgangsstellung.

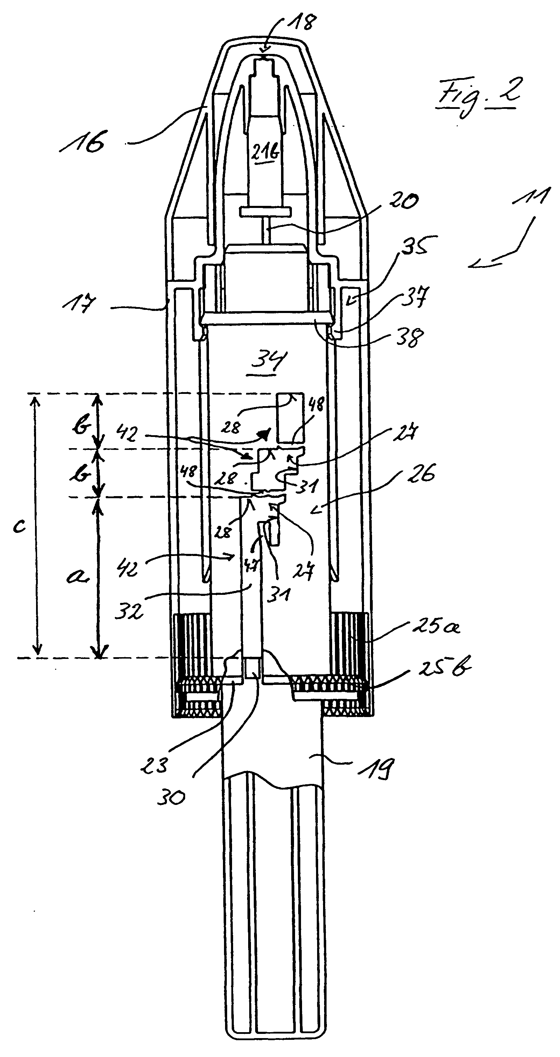

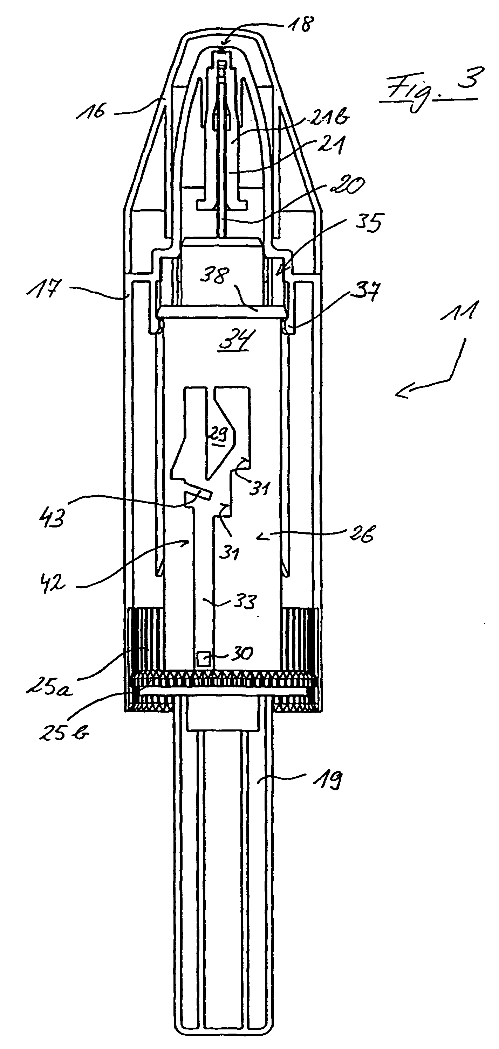

Dabei ist in Fig. 1 eine Schnittdarstellung durch die erfindungsgemäße Austragvorrichtung dargestellt. Die Austragvorrichtung 11 weist ein Gehäuse 17 mit einer Austragöffnung 18 zum Austragen des Mediums auf. In dem Gehäuse ist eine Nadel 20, beispielsweise mittels einem Füllstück 21b, angeordnet, die geeignet ist, den Austragkanal 21 zwischen erster Kammer 39 des Behälters 12 und der Austragöffnung 18 zu bilden. An dem Gehäuse 17 sind nach innen abragende Rastnasen 37 ausgebildet, die den Rastring 38 der Hülse 34 hintergreifen können und so dazu geeignet sind, eine definierte Austragstellung der Hülse 37 in dem Gehäuse 17 festzulegen. Des weiteren ist in dem Gehäuse 17 auch das Betätigungsmittel 19 angeordnet. Es handelt sich dabei um aus zwei hohlzylindrischen Elementen bestehenden, konzentrisch angeordneten Rohrabschnitten, die an ihrem unteren Ende mittels einer gemeinsamen Betätigungsfläche abgeschlossen werden. Die Betätigungsfläche ist im wesentlichen radial zur Erstreckung der Rohrabschnitte ausgebildet und manuell betätigbar. Die Rohrabschnitte weisen unterschiedliche Längen auf. Der innere Rohrabschnitt ist vom Durchmesser her so ausgebildet, daß er in den Behälter 12 einführbar ist. Der äußere Rohrabschnitt, dessen Axialerstrekung vorzugsweise länger ist als die des inneren Rohrabschnittes, gleitet entlang der Hülse 34. Zwischen Hülse 34 und Betätigungsmittel 19 ist eine Kulissenführung ausgebildet, die in den Fign. 2 und 3 dargestellt ist.

In dem Gehäuse 17 ist eine Hülse 34 angeordnet, die als Aufnahme für den Behälter 12 für das Medium dient. Soweit der Behälter 12 an seinem vorderen Ende mit einem Crimp-Verschluß 22 verschlossen ist, kann in einfacher Weise der Behälter 12 dadurch in der Hülse 34 befestigt werden, das an der Hülse 34 eine Verrastung für die Crimp-Hülse 22 ausgebildet ist. Alternativ, bzw. ergänzend hierzu ist es auch möglich, in der Hülse 34 Rippen 49 vorzusehen, die in Richtung des Behälters 12 nach innen abragen und den Behälter 12 zumindest in dessen oberen Bereich von beispielsweise 5mm bis 10mm klemmend halten. Vorzugsweise sind hierfür drei Rippen 49 vorgesehen. Auf diese Weise ist es möglich, den Behälter 12 lagefest in der Hülse 34 zu halten. Daneben erstreckt sich die Hülse auch über wenigstens einen Teil der axialen Länge des Behälters 12. In der dargestellten Ausführungsform erstreckt sich die Hülse 34 bis nahezu an das hintere Ende des Gehäuses 17. Damit eine verdrehsichere Lagerung der Hülse 34 in dem Gehäuse 17 sichergestellt ist, weist das Gehäuse 17 eine sich an einem axialen Abschnitt erstreckende Verzahnung 25a an seiner Innenseite auf. In diese Verzahnung 25a am Gehäuse greift eine an der Hülse ausgebildete Verzahnung 25b ein. Die Verzahnungen 25a, 25b verhindern durch ihren gegenseitigen Eingriff, daß die Hülse 34 gegenüber dem Gehäuse 17 verdreht wird. Gleichzeitig ist es jedoch möglich, daß die Hülse 34 im Gehäuse 17 eine axiale Bewegung durchführt. Dabei müssen die Längen der Verzahnungen so ausgebildet sein, daß die Hülse 34 von der dargestellten Ausgangsstellung 35 in die Austragstellung verbringbar ist und die Verzahnungen dennoch in gegenseitigem Eingriff verbleiben und nicht anstehen.

In der Hülse 34 ist der Behälter 12 angeordnet. Der Behälter 12 wird beispielsweise über die Crimp-Hülse des Crimp-Verschlusses 22 in der Hülse 34 gehalten. Bei dem Behälter 12 handelt es sich vorzugsweise um einen Glas-Behälter. Derartige Behälter sind beispielsweise Kapsulen oder auch Karpulen. Anstelle von Glas-Behältern können auch Behälter aus einem Kunststoff verwendet werden. Der Behälter muß lediglich den Anforderungen an Dichtheit, Sterilität und Stabilität sowie Festigkeit genügen. Ferner sollte er einfach herzustellen sein. Der Behälter 12 weist zwei Kammern, nämlich eine erste Kammer 13a und weitere Kammer 13b auf. Die beiden Kammern sind durch einen Stopfen 14 voneinander getrennt. Dabei ist der Stopfen in der dargestellten Ausgangsstellung in einer Stellung, in der er die beiden Kammern vollständig voneinander trennt. Der Stopfen 14 ist dabei axial in dem Behälter 12 verschiebbar. Er kann in eine Stellung verbracht werden, wo er sich im Bereich des Überströmkanales 15 befindet. In diesem Fall ist dann eine Verbindung zwischen den beiden Kammern 13a, 13b hergestellt. Der Überströmkanal 15, der insbesondere als By-pass ausgebildet ist, weist hierzu eine Länge auf, die etwas größer ist als die axiale Länge des Stopfens 14. An dem vorderen, der Austragöffnung 18 der Austragvorrichtung 11 zugewandten Seite ist der Behälter 12 durch einen Crimp-Verschluß 22 verschlossen. Bei einem derartigen Crimp-Verschluß wird ein Stopfen, der dichtend in der Öffnung des Behälters 12 platziert ist, durch einen Crimp-Ring gehalten und teilweise auf den Behälter 12 aufgepreßt. Der Crimp-Ring wiederum ist an einer Überwurfkante des Behälters 12, die zu diesem Zweck ausgebildet ist, gehalten. Hierbei wird als Stopfen meist ein gummielastisches Material verwendet, so daß der Stopfen den Crimp-Ring gegenüber der Überwurfkante verspannt. Hierdurch ist es möglich, einen festen, gut dichtenden Verschluß des Behälters 12 zu schaffen. An seinem hinteren Ende wird die letzte weitere Kammer 13b ebenfalls durch einen Stopfen 14 verschlossen. Wie der Stopfen 14, der die beiden Kammern voneinander trennt, ist auch dieser Stopfen 14 axial beweglich in dem Behälter 12 angeordnet. Es wird jedoch durch den Stopfen 14 ein hermetischer Verschluß der Kammer gewährleistet. Auf diesen Stopfen 14 kann der innere Rohrabschnitt des Betätigungsmittels 19 einwirken und zu diesem Zweck auch schon im unbetätigten, in der Fig. 1 dargestellten Ausgangsstellung, an diesem Stopfen 14 anliegen.

In der ersten Kammer 13a ist eine Medienkomponente, beispielsweise das Lyophilisat 40, eingebracht. Dabei ist genau diese Menge an Medienkomponente in die erste Kammer eingebracht worden, die für den Austrag von der erwünschten Anzahl von Teilchargen erforderlich ist. Eine sehr genaue Dosierung der Menge der Medienkomponente ist möglich. Daneben beinhaltet die erste Kammer 13a auch eine Gasblase 39. Die Gasblase kann insbesondere durch ein Inertgas, durch Reinraumluft oder durch normale Umgebungsluft gebildet werden. Es kann vorteilhaft sein, die befüllte Karpule und damit auch das Gas anschließend zu sterilisieren. Das Gas sollte möglichst frei von jeder Art von Fremdstoffen und Verunreinigungen sein. Insbesondere ist dabei darauf zu achten, daß das Gas der Gasblase nicht mit der in die Kammer 13a eingebrachten Medienkomponente in einer Weise reagiert, die deren Wirksamkeit oder Haltbarkeit stört.

In den weiteren Kammern 13b, wobei im dargestellten Ausführungsbeispiel lediglich eine weitere Kammer 13b vorhanden ist, sind die restlichen Medienkomponenten untergebracht. In dem dargestellten Beispiel ist ein Trägermedium 41 in die weitere Kammer 13b eingebracht. Bei dem Trägermedium 41 handelt es sich insbesondere um ein Lösungsmittel für das Lyophilisat 40. Vorzugsweise wird die weitere Kammer 13b möglichst vollständig mit dem Trägermedium 41 gefüllt. Es handelt sich dabei gemäß dem dargestellten Beispiel möglichst um eine nicht oder nur wenig kompressible Flüssigkeit. Auch hier ist darauf zu achten, daß die Medienkomponente nicht durch etwa vorhandene Restluft oder andere Einbringungen oder Verschmutzungen im Behälter verunreinigt ist. Dies kann aber mit üblichen Herstellungsmethoden und Befüllmethoden sichergestellt werden. Als Trägermedium 41 kommen dabei bevorzugt Wasser bzw. wässrige Lösungen, wie beispielsweise Alkohol enthaltende Lösungen, in Frage. Auf Wasserbasis hergestellte Trägermedien 41 sind in der Regel geeignet, das Lyophilisat 40 zu lösen bzw. so aufzunehmen, daß eine gleichmäßige Durchmengung des Trägermediums mit dem Lyophilisat 40 oder dem anderen, in die erste Kammer 13a eingebrachte Medienkomponente gewährleistet ist.

Die Fign. 2 und 3 zeigen, jeweils in teilgeschnittener Darstellung, die Austragvorrichtung der Fig. 1 von zwei einander gegenüberliegenden Seiten. Dabei ist im Unterschied zur Fig. 1 die Hülse 34 im wesentlichen ungeschnitten dargestellt, so daß die Ausbildung der Kulissenführung 26 zwischen dem Betätigungsmittel 19 und der Hülse 34 sichtbar ist.

Gemäß der Darstellung der Fign. 2 und 3 ist die Austragvorrichtung 11 mit einer Schutzkappe 16 verschlossen. Diese Schutzkappe 16 ist abnehmbar. Sie dient dem Schutz der Austragöffnung 18 vor Verschmutzung. Vorzugsweise ist die Schutzkappe 16 so ausgebildet, daß sie gleichzeitig die Funktion einer Kindersicherung übernimmt. Dies geschieht beispielsweise dadurch, daß die Schutzkappe an bestimmten Stellen gegriffen werden muß und leicht zusammengedrückt werden muß, damit sie von dem Gehäuse 18 abziehbar ist. Gleichzeitig kann vorgesehen sein, daß zwischen der Schutzkappe 16 und dem Betätigungsmittel 19 oder der Hülse 34 eine Betätigungssperre wirkt. Die Betätigungssperre soll ein Betätigen des Betätigungselementes 19 verhindern, soweit und solange die Schutzkappe 16 auf die Austragvorrichtung 11 aufgesetzt ist.

Die Hülse 34 ist in dem Gehäuse 17 der Austragvorrichtung 11 gehalten. Sie befindet sich in der Ausgangsstellung 35. In der Ausgangsstellung 35 ist die Hülse 34 durch die Rastnasen 37 gehalten, die den Rastring 38 hintergreifen. Dabei ist die Hülse 34 axial in Richtung auf die Austragöffnung 18 verschiebbar und gelangt am Ende dieses Verschiebeweges in die nicht dargestellte Austragstellung. Über die Verzahnung 25a, 25b an der Innenseite des Gehäuses 17 bzw. an der Hülse 34 ist sichergestellt, daß die Hülse 34 nicht in dem Gehäuse 17 verdrehbar ist. Zwischen dem Betätigungsmittel 19, das ebenfalls in dem Gehäuse 17, bzw. in der Hülse 34, geführt gehalten ist, wobei die Führung vorzugsweise durch die Hülse 34 und den Behälter 12 erfolgt, ist eine Kulissenführung 26 ausgebildet. Die Kulissenführung wird durch Kulissenbahnen 32, 33 - die gemäß dem dargestellten Ausführungsbeispiel in der Hülse 34 ausgebildet sind - und darin geführten Gleitsteinen 30, - die an dem Betätigungsmittel 19 ausgeformt sind - gebildet.

In der Hülse 34 ist eine erste Kulissenbahn 32 und auf der gegenüberliegenden, in Fig. 3 dargestellten Seite, eine zweite Kulissenbahn 33 ausgebildet. Die beiden Kulissenbahnen haben unterschiedliche Funktionen. Die erste Kulissenbahn 32 dient der Unterteilung des Betätigungsweges **c** des Betätigungsmittels 19 in den ersten Teilbetätigungsweg **a** und die nachfolgenden Teilbetätigungswege **b**. Aufgabe der zweiten Kulissenbahn 33 ist es im wesentlichen, die Durchführung der selbsttätigen Umschaltbetätigung zwischen den weiteren Teilbetätigungen durchzuführen. In dem dargestellten Ausführungsbeispiel ist es vorgesehen, daß die Umschaltbetätigung zwischen der ersten Teilbetätigung **a** und der ersten nachfolgenden Teilbetätigung **b** manuell und nicht selbsttätig erfolgt. Durch eine entsprechende Ausbildung der zweiten Kulissenbahn 33 wäre es aber auch möglich, daß diese Umschaltbetätigung ebenfalls selbsttätig erfolgt. Ein Vorteil einer manuellen Umschaltbetätigung zu diesem Zeitpunkt ist darin zu sehen, daß es den Benutzer daran erinnern kann, daß es zwecks einer guten Durchmischung zwischen den Medienkomponenten zur Bildung des auszutragenden Mediums erforderlich oder wünschenswert sein kann, wenn ein Schütteln der Austragvorrichtung 11 erfolgt, bevor die nachfolgenden Teilbetätigungen durchgeführt werden, denen ein Austrag von Medium zugeordnet ist.

Die erste Kulissenbahn 32, in der der betätigungsmittelseitige Gleitstein 30 geführt ist, unterteilt den Betätigungsweg **c** des Betätigungsmittels 19 in einen ersten Teilbetätigungsweg **a** und mehrere nachfolgende Teilbetätigungswege **b**, wobei der erste Teilbetätigungsweg der ersten Teilbetätigung und die nachfolgenden Teilbetätigungswege **b** jeweils einer nachfolgenden Teilbetätigung zugeordnet sind. Die Unterteilung des Betätigungsweges **c** in die Teilbetätigungswege erfolgt dabei durch jeweils einen Anschlag 28, der am Ende einer dem Teilbetätigungsweg zugeordneten linearen Führungsabschnitt 42 ausgebildet ist. Hierzu sind die linearen Führungsabschnitte 42 stufenförmig zueinander versetzt. In dem Bereich des jeweiligen Anschlages 28 ist dabei so ein breiter Abschnitt ausgebildet, daß der Gleitstein 30 durch Verdrehen des Betätigungsmittels 19 bzgl. der Hülse 34 und somit dem Gehäuse 17 auf den nächstfolgenden linearen Führungsabschnitt 42 ausgerichtet werden kann. Der Verdrehweg wird dabei jeweils von einem Anschlag 31 begrenzt. Zu Beginn zumindest jedes nachfolgenden Teilbetätigungsweges **b** sind auf den Gleitstein 30 einwirkende Druckpunktmittel 27 ausgebildet. Die Druckpunktmittel stellen sicher, daß zu Beginn des Betätigungsvorganges die Betätigungskraft des Betätigungsmittels 19 einen Schwellenwert überschreitet, so daß die vollständige Durchführung einer Teilbetätigung jeweils gewährleistet ist. In der dargestellten Ausführungsform sind hierzu zu Beginn der Teilbetätigungswege Materialstege 48 ausgebildet, die zumindest eine Sollbruchstelle aufweisen und bei Überschreiten der Mindestkraft durchbrochen werden. Um sicherzustellen, daß zu Beginn der Durchführung der ersten Teilbetätigung eine für diese ausreichende Betätigungskraft auf das Betätigungsmittel 19 einwirkt, kann es vorgesehen sein, daß, wie in Fig. 1 dargestellt, ein Haltering 23 am Gehäuse oder an der Hülse 34 angeordnet ist, welcher dort abgestützt ist und über Sollbruchstellen 24 mit dem Betätigungsmittel 19 verbunden sind. Damit kann die erste Teilbetätigung erst dann erfolgen, wenn die für die erste Teilbetätigung erforderliche Mindestkraft überschritten wird.

Die in der Fig. 3 dargestellte zweite Kulissenbahn 33, in der ebenfalls ein mit dem Betätigungsmittel 19 verbundener Gleitstein 30 geführt wird, hat eine andere Aufgabe. Während der ersten Teilbetätigung wird lediglich der Gleitstein 30 in dem linearen Führungsabschnitt 42 geführt.

Nach Durchführung der ersten Umschaltbetätigung, also der Umschaltbetätigung zwischen dem ersten Teilbetätigungsweg und dem ersten nachfolgenden Teilbetätigungsweg, dient die in die Kulissenbahn hineinragende Rückdrehsperre 43 dazu, zu verhindern, daß ein Zurückdrehen des Betätigungsmittels 19 aus der Ausgangslage für die erste nachfolgende Teilbetätigung erfolgt. Dabei ist in der zweiten Kulissenbahn 33 jeweils ein Anschlag 31 ausgebildet, der als Endlagendefinition für die Umschaltbetätigung dient. Während den nachfolgenden Teilbetätigungen wird ein in den linearen Führungsabschnitt hineinragender Kraftspeicher 29 aus dem Führungsabschnitt verdrängt. Dabei wird die Verformungsenergie der elastischen Verformung des Kraftspeichers 29 zur Durchführung der nachfolgenden Umschaltbetätigung genutzt. Für die Erzeugung der Vorspannung und die Sicherung vor einem vorzeitigen Durchführen der Umschaltbetätigung vor Erreichen der Endlage der jeweiligen Teilbetätigung dient die in der Fig. 2 dargestellte erste Kulissenbahn 32.

Aus der in den Fign. 1 bis 3 dargestellten Ausgangsstellung heraus ist auch die Vorgehensweise dem Ablauf einer vollständigen Betätigung der Austragvorrichtung erläutert.

Zu Beginn der Betätigung muß die Schutzkappe 16 abgenommen werden. Dadurch kann auch eine Betätigungssperre für das Betätigungselement 19 aufgehoben werden. Gleichzeitig kann ein Lagesicherungsschutz für die Beibehaltung der Ausgangsstellung 35 für die Hülse 34 mit dem darin gehaltenen Behälter 12 aufgehoben werden, so daß der Behälter 12 zusammen mit der Hülse 34 in die durch die Rastnasen 37 definierte Austragstellung verbringbar ist.

Damit ein Vermischen der Medienkomponenten der ersten Kammer 13a mit der Medienkomponente der weiteren Kammer 13b, also dem Trägermedium 41, erfolgen kann, muß das Betätigungsmittel 19 kraftbeaufschlagt in Richtung der Austragöffnung 18 in das Gehäuse 17 hineinbewegt werden. Dabei reißen die Sollbruchstellen 24 zwischen dem Betätigungsmittel 19 und dem Haltering 23. Die Gleitsteine 30 gleiten in der Kulissenführung 26 mit den beiden Kulissenbahnen 32, 33. Aufgrund der erforderlichen Mindestbetätigungskraft zum Überwinden der Bruchkräfte der Sollbruchstelle 24 erfolgt eine vollständige erste Teilbetätigung über den gesamten ersten Teilbetätigungsweg **a** bis zum Erreichen des Anschlages 28 am Ende des ersten linearen Führungsabschnittes 42 der ersten Kulissenbahn 32. Durch die Rückzugsperre 47 der ersten Kulissenbahn 32 wird ein Zurückziehen oder Zurückschieben des Betätigungselementes 19 verhindert. Während dieser Hubbewegung wird der am Ende des Behälters 12 angeordnete Stopfen 14, der in Kraftschluß mit dem Betätigungsmittel 19 ist, in Richtung auf die Austragöffnung 18 geschoben. Aufgrund der sich in der weiteren Kammer 13b befindlichen inkompressiblen Flüssigkeit - dem Trägermedium 41 - wird dabei der mittlere Stopfen 14, der die erste Kammer 13a von der weiteren Kammer 13b trennt, ebenfalls in Richtung auf die Austragöffnung 18 nach vorne geschoben. Diese Bewegung des Stopfens 14 zwischen den beiden Kammern 13a, 13b erfolgt solange, bis das Trägermedium 41 an diesem Stopfen 14 vorbei durch den Überströmkanal 15 in die erste Kammer 13a fließen kann. Gleichzeitig mit der Bewegung des Stopfens 14 zwischen den beiden Kammern 13a, 13b wird das Lyophilisat 40, das sich in der ersten Kammer 13a befindet, nach vorne, also in Richtung auf die Austragöffnung 18 geschoben. Durch das einströmende Trägermedium 41 wird das Lyophilisat 40 langsam aufgelöst. Aus den beiden Medienkomponenten entsteht austragbares Medium.

Während dieser ersten Teilbetätigung über den Teilbetätigungsweg **a** hinweg entsteht in der ersten Kammer 13a ein Überdruck, da das Gesamtaufnahmevolumen des Behälters 12 ständig reduziert wird. In die erste Kammer 13a strömt das Trägermedium 41 der weiteren Kammer 13b, deren eigenes Volumen langsam auf Null reduziert wird. Dadurch wird in der Gasblase 39 der ersten Kammer 13a ein stetig steigender Druck aufgebaut. Dieser Druck wird so groß, daß er der weiteren Bewegung der Stopfen 14 in dem Behälter 12 entgegenwirkt. Dadurch entsteht ein Kraftschluß zwischen dem Betätigungsmittel 19 und der Hülse 34, wodurch der gesamte Behälter 12 mitsamt der Hülse 34, in der der Behälter 12 lagefest gehalten ist, in Richtung auf die Austragöffnung 18 nach vorne geschoben wird. Der sich in dem Crimp-Verschluß 22 befindliche Stopfen wird dabei langsam von der Spitze der Nadel 20 durchstochen. Sobald über die Nadel 20 der Verschluß des Behälters vollständig durchstochen wurde und somit über den Austragkanal 21 der Nadel 20 eine fluidische Verbindung zwischen der ersten Kammer 13a und der Austragöffnung 18 hergestellt ist, kann der Überdruck aus der ersten Kammer 13a entweichen, der Kraftschluß zwischen Hülse 34 und Betätigungsmittel 19 ist dann unterbrochen. Der Gleitstein 30, der in der ersten Kulissenbahn 32 geführt ist, gelangt in Anlage mit dem Anschlag 28. Er wird nun weiter gedrückt und dadurch die Hülse 34 zusammen mit dem darin gehaltenen Behälter 12 weiter nach vorne geschoben, bis die Rastnasen 37 den Rastring 38 hintergreifen. In dieser Stellung ist die Austragstellung der Hülse 34 und des darin gehaltenen Behälters 12 erreicht. Die Hülse 34 ist nun lagefest bzgl. dem Gehäuse 17 der Austragvorrichtung. Ein Zurückziehen der Hülse 34 ist dadurch wirkungsvoll verhindert. Aus der ersten Kammer 13a ist noch kein Medium ausgetragen worden. Lediglich das Gas der Gasblase wurde verdrängt.

Nachdem die Rückzugsperre 47 in dieser Lage den Gleitstein 30 der ersten Kulissenbahn 32 gegen ein Zurückziehen sichert, kann in dieser Stellung zum Erzeugen des Mediums und zur guten Durchmischung bzw. der guten Auflösung der Medienkomponenten miteinander bzw. ineinander der Behälter 17 geschüttelt werden. In dieser Stellung ist der hintere Stopfen 14 vollständig zum vorderen Stopfen 14 hin bewegt, so daß das Volumen der weiteren Kammer 13b auf Null reduziert und das gesamte Trägermedium 41 in die erste Kammer 13a verdrängt wurde.

Um die erste nachfolgende Teilbetätigung, also den ersten Austrag von Medium durch die Austragöffnung 18 durchzuführen, muß nun zunächst die Austragvorrichtung an den Applikationsort eingesetzt werden. Beispielweise muß das Gehäuse 17 mit der Austragöffnung 18 in eines der beiden Nasenlöcher eines Patienten eingeführt werden. Nun oder auch vor dem Ansetzen der Austragvorrichtung am Patienten muß das Betätigungsmittel 19 im Uhrzeigersinn bis zum Anschlag 31 der beiden Kulissenbahnen 32, 33 der Kulissenführung 26 verdreht werden. Ein Rückdrehen wird nach Vollzug dieser Umschaltbetätigung durch die Rückdrehsperre 43 der zweiten Kulissenbahn 33 verhindert. Dabei verhindert die Verzahnung 25a, 25b zwischen Hülse 34 und Gehäuse 17 ein versehentliches Verdrehen der Hülse 34 in dem Gehäuse 17. Die erste nachfolgende Teilbetätigung, über den nachfolgenden Teilbetätigungsweg **b** hinweg, erfolgt nun durch Aufbringen einer Betätigungskraft am Betätigungsmittel 19, die an ihrem Boden angreift und in Richtung auf die Austragöffnung 18 gerichtet ist. Dabei muß das Druckpunktmittel 27 der ersten Kulissenbahn, es handelt sich dabei um einen Materialsteg 48, der am Beginn des zugehörigen linearen Führungsabschnittes 42 ausgebildet ist, überwunden werden. Das Druckpunktmittel weist dabei vorzugsweise Sollbruchstellen auf. Danach ist sichergestellt, daß eine Betätigung durchgeführt wird, die sich über den gesamten Teilbetätigungsweg **b** der nachfolgenden Teilbetätigung erstreckt und die wiederum durch den entsprechenden Anschlag 28 in der ersten Kulissenbahn 32 definiert wird. Während dieser Bewegung wird auf der gegenüberliegenden Seite in der zweiten Kulissenbahn der Kraftspeicher 29 zur Seite gedrückt. Durch die elastischer Verformung des Kraftspeichers 29 wird eine Vorspannung des Kraftspeichers erzeugt, die der nachfolgenden Umschaltbetätigung, die vor der zweiten nachfolgenden Teilbetätigung erfolgt, dient.

Während der ersten nachfolgenden Teilbetätigung wird eine entsprechende Menge Medium, das aus beiden Medienkomponenten gebildet ist, durch den Austragkanal 21 der Nadel 20 aus der ersten Kammer 13a verdrängt und gelangt zur Austragöffnung 18. Dort wird das Medium zerstäubt und ausgetragen.

Vor Durchführung der zweiten, letzten nachfolgenden Teilbetätigung muß die Betätigungskraft auf das Betätigungsmittel 19 abgesenkt werden. Damit wird der Anpreßdruck des Gleisteins 30 auf den Anschlag 28 verringert. Der vorgespannte Kraftspeicher 29 dreht nun die Hülse 34 weiter, bis die Gleitsteine 30 den nächsten Anschlag 31 erreichen. Der Kraftspeicher 29 ist dann entspannt, der Gleitstein 30 der ersten Kulissenbahn 32 befindet sich unmittelbar vor den zweiten Druckpunktmitteln 27 und dem letzten linearen Führungsabschnitt 42. Der zweite Anwendungshub erfolgt durch nochmaliges Betätigen des Betätigungsmittels 19 in gleicher Weise und mit gleicher Wirkung wie bei der ersten nachfolgenden Teilbetätigung. Auch diesmal wird wiederum ein Druckpunktmittel 27 zerstört. Der Austrag des Mediums aus der ersten Kammer 13a wird durch weiteres Verschieben der beiden Stopfen 14 des Behälters 12 in Richtung auf die Austragöffnung 18 erreicht. Das Medienvolumen, das ausgetragen wird, wird dabei durch den Betätigungsweg der nachfolgenden Teilbetätigung, hier sowohl bei der ersten Teilbetätigung wie auch bei der zweiten Teilbetätigung der Teilbetätigungsweg **b**, bestimmt. Der Behälter, eine Ampulle, Kapsule oder Karpule ist nun entleert. Relevante Teile der Austragvorrichtung sind nach der Anwendung zerstört und können nicht wieder verwendet werden. Es handelt sich also um ein Einweg-System und kann anschließend dem Recycling zugeführt werden. Dabei ist es vorteilhaft, daß die Austragvorrichtung überwiegend aus Kunststoff herstellbar ist, der entsprechend sortenrein gewählt werden kann. Gemäß bevorzugter Ausgestaltung sind lediglich der Crimp-Verschluß und die Nadel als metallische Teile ausgebildet, wobei gegebenenfalls auch der Crimp-Verschluß aus Kunststoff hergestellt ist. Die Stopfen 14 sind häufig aus einem Elastomer oder Gummi. Auch sie können einem Recycling zugeführt werden. Besonders vorteilhaft ist also bei einer derartigen Ausbildung der Austragvorrichtung die geringe Verwendung unterschiedlicher Materialien und die geringe Anzahl von metallischen Bauteilen. Es sind keine metallischen Federn, Stifte oder andere metallische Elemente, außer den vorstehend genannten, erforderlich. Nach dem Abnehmen der Schutzkappe ist die Austragvorrichtung bereit für den Mischvorgang der Medienkomponente, also zu der Durchführung der ersten Teilbetätigung. Danach muß das Betätigungsmittel im Uhrzeigersinn weitergedreht werden. Dieses manuelle Weiterdrehen kann auch durch ein selbsttätiges Weiterdrehen ersetzt werden. Anschließend ist die Austragvorrichtung bereit zur Anwendung. Die nachfolgenden Teilbetätigungen, die dem Austrag von Medium dienen, können direkt nacheinander und ohne irgend welche zusätzliche Handbewegungen ausgeführt werden. Die Handhabe der Austragvorrichtung ist also trotz der erforderlichen Mischung des Mediums aus den Medienkomponenten einfach durchzuführen. Ein wesentlicher Vorteil der Erfindung ist in dieser einfachen Handhabbarkeit zu sehen.

## Patentansprüche

1. Austragvorrichtung, insbesondere für wenigstens einen pharmazeutischen Wirkstoff enthaltenden Medien, mit einem Behälter (12), in dem das auszutragende Medium bevorratet ist, wobei der Behälter (12) voneinander getrennte Kammern (13a, 13b) aufweist, in denen jeweils eine Medienkomponente (40, 41) enthalten ist, die zusammen das auszutragende Medium ergeben, wobei der Behälter (12) in einem Gehäuse (17) angeordnet ist, das eine Austragöffnung (18) zum Austrag von Medium aufweist, wobei ein bezüglich dem Gehäuse (17) relativ bewegliches Betätigungsmittel (19) vorgesehen ist, dessen Betätigung zunächst eine Verbindung zwischen den Kammern des Behälters herstellt, wobei der Betätigungsweg (c) des Betätigungsmittels (19) in einen ersten Teilbetätigungsweg (a) und eine Anzahl nachfolgender Teilbetätigungswege (b) unterteilt ist, wobei während des ersten Teilbetätigungsweges (a) ein Vermischen der Medienkomponenten (40, 41) zu dem auszutragenden Medium erfolgt und wobei jeder nachfolgenden Teilbetätigung des Betätigungsmittels (19) der Austrag einer definierten Teilcharge des Mediums zugeordnet ist, **dadurch gekennzeichnet, dass** der Behälter (12) in dem Gehäuse (17) von einer Ausgangsstellung, in der der Behälter (12) hermetisch verschlossen ist, infolge der Betätigung des Betätigungsmittels (19) in eine Austragstellung verschiebbar angeordnet ist, in der durch die Verschiebung eine fluidische Verbindung zwischen dem Behälter (12) und der Austragöffnung (18) hergestellt ist, und dass die Austragöffnung (18) zu einem zerstäubten Austrag des Mediums ausgebildet ist.

2. Austragvorrichtung nach Anspruch 1, **dadurch gekennzeichnet, dass** die Kammern (13a, 13b) mittels im Behälter (12) angeordneter Stopfen (14) voneinander getrennt sind.

3. Austragvorrichtung nach Anspruch 1 oder 2, **dadurch gekennzeichnet, daß** der Behälter (12) Überströmkanäle (15) aufweist, wobei während der ersten Teilbetätigung (a) die Kammern (13a, 13b) voneinander trennende Stopfen (14) in eine Position im Bereich von Überströmkanälen (15) verbringbar sind, so daß über die Überströmkanäle (15) die Kammern miteinander verbunden sind.

4. Austragvorrichtung nach einem der vorhergehenden Ansprüche, **dadurch gekennzeichnet, daß** eine Kulissenführung (26) zur Unterteilung des Betätigungsweges (c) in die Teilbetätigungswege (a, b) vorgesehen ist.

5. Austragvorrichtung nach Anspruch 4, **dadurch gekennzeichnet, daß** die Kulissenführung (26) Druckpunktmittel (27) aufweist, wobei Druckpunktmittel (27) zu Beginn jeder nachfolgenden Teilbetätigung zu überwinden sind.

6. Austragvorrichtung nach einem der Ansprüche 4 oder 5, **dadurch gekennzeichnet, daß** die Kulissenführung (26) für jede Teilbetätigung einen linearen Führungsabschnitt (42) aufweist, wobei die Führungsabschnitte (42) derart voneinander versetzt sind, daß sie jeweils durch einen Anschlag (28) begrenzt sind und zwischen zwei Teilbetätigungen jeweils eine von der Teilbetätigung verschiedene Umschaltbetätigung durchzuführen ist.

7. Austragvorrichtung nach Anspruch 6, **dadurch gekennzeichnet, daß** wenigstens die Umschaltbetätigungen zwischen zwei nachfolgenden Teilbetätigungen (b) selbsttätig durchgeführt werden.

8. Austragvorrichtung nach Anspruch 7, **dadurch gekennzeichnet, daß** Kraftspeicher (29) vorgesehen sind, die während der Durchführung einer Teilbetätigung vorgespannt werden und der darauffolgenden selbsttätigen Durchführung der Umschaltbetätigung dienen.

9. Austragvorrichtung nach einem der vorhergehenden Ansprüche, **dadurch gekennzeichnet, daß** der Behälter (12) in einer Hülse (34) lagefest angeordnet ist, wobei die Hülse (34) zum Gehäuse (17) relativ beweglich ist und von der Ausgangsstellung (35) in die Austragstellung verschiebbar ist, wobei in der Austragstellung die fluidische Verbindung zwischen der ersten Kammer (13a) des Behälters und der Austragöffnung (18) hergestellt ist.

10. Austragvorrichtung nach Anspruch 9, **dadurch gekennzeichnet, daß** das Verbringen der Hülse (34) von der Ausgangsstellung (35) in die Austragstellung während der ersten Teilbetätigung (a) erfolgt, wobei zur Herstellung der fluidischen Verbindung zwischen Austragöffnung (18) und der ersten Kammer (13a) des Behälters (12) eine gehäuseseitige, einen Austragkanal (21) aufweisende Nadel (20) einen behälterseitigen, vorzugsweise mit einem Crimp-Verschluß (22) gehaltenen Stopfen durchsticht.

11. Austragvorrichtung nach Anspruch 9 oder 10, **dadurch gekennzeichnet, daß** die Kulissenführung (26) zwischen Hülse (34) und Betätigungsmittel (19) ausgebildet ist und vorzugsweise der wenigstens eine Gleitstein (30) an dem Betätigungsmittel (19) und die wenigstens eine Kulissenbahn (32, 33) der Kulissenführung (26) an der Hülse (34) ausgebildet ist.

12. Austragvorrichtung nach einem der Ansprüche 6 bis 11, **dadurch gekennzeichnet, daß** die Umschaltbetätigung zwischen der ersten Teilbetätigung (a) und der ersten nachfolgenden Teilbetätigung (b) durch ein Verdrehen des Betätigungsmittels gegenüber dem Gehäuse (12) erfolgt, wobei der Drehwinkel durch Anschlagkanten (31) der Kulissenführung (26) vorgegeben ist und wobei vorzugsweise mittels einer Verzahnung (25a, 25b) ein Verdrehen der Hülse (34) gegenüber dem Gehäuse (17) gesperrt ist.

13. Austragvorrichtung nach einem der vorhergehenden Ansprüche, **dadurch gekennzeichnet, daß** der Behälter (12) eine erste, der Austragöffnung (18) der Austragvorrichtung (11) zugewandten Kammer (13a) und wenigstens eine weitere Kammer (13b) aufweist, die mit dem im Behälter axial verschiebbaren Stopfen (14), welche die Kammern voneinander trennen und verschließen und mit einem die letzte der Kammern (13b) verschließenden Stopfen (14), auf dem das Betätigungsmittel (19) einwirkt, versehen sind.

14. Austragvorrichtung nach einem der vorhergehenden Ansprüche, **dadurch gekennzeichnet, daß** die erste, der Austragöffnung (18) zugewandten Kammer (13a) ein Volumen mit einer Gasblase (39) aufweist, wobei während der ersten Teilbetätigung (a) durch Kompression der Gasblase (39) ein Kraftschluß zwischen Betätigungsmittel (19) und Behälter (12) herstellbar ist, mittels dem der Behälter (12) von der Ausgangsstellung (35), in der der Behälter (12) hermetisch verschlossen ist, in die Austragstellung, in der eine fluidische Verbindung zwischen Austragöffnung (18) und erster Kammer (13a) hergestellt ist, verbringbar ist.

15. Austragvorrichtung nach einem der Ansprüche 1 bis 14, **dadurch gekennzeichnet, daß** der Behälter (12) zwei Kammern (13a, 13b) aufweist, wobei in die erste Kammer (13a) vorzugsweise eine trockene, insbesondere lösliche Medienkomponente, vorzugsweise ein Lyophilisat (40), eingebracht ist und wobei in der zweiten Kammer (13b) ein Trägermedium (41), vorzugsweise ein insbesondere flüssiges Lösungsmittel, als Medienkomponente eingebracht ist.

16. Austragvorrichtung nach einem der vorhergehenden Ansprüche, **dadurch gekennzeichnet, daß** zwei insbesondere einen gleich großen Austrag bewirkende nachfolgende Teilbetätigungen (b) durchführbar sind.

## Claims

1. Discharge apparatus, particularly for media containing at least one pharmaceutical substance, having a container (12) storing the medium to be discharged, the container (12) having separate chambers (13a, 13b), each of which contains a media component (40, 41), which together provide the medium to be discharged, the container (12) being located in a casing (17) having a discharge opening (18) for discharging the medium, an actuating means (19) movable relative to the casing (17) being provided and whose actuation initially produces a connection between the chambers of the container, the actuating path (c) of the actuating means (19) being subdivided into a first partial actuating path (a) and a plurality of successive partial actuating paths (b) and during the first partial actuating path (a) there is a mixing of the media components (40, 41) to give the medium to be discharged and with each successive partial actuation of the actuating medium (19) is associated the discharge of a clearly defined partial medium charge, **characterized in that** in the casing (17) the container (12) is displaceable from a starting position where the container (12) is hermetically sealed into a discharge position as a result of the actuation of the actuating means (19), in which as a result of the displacement a fluidic connection is produced between the container (12) and the discharge opening (18) and that the discharge opening (18) is constructed for an atomized discharge of the medium.

2. Discharge apparatus according to claim 1, **characterized in that** the chambers (13a, 13b) are separated from one another by means of plugs (14) located in the container (12).

3. Discharge apparatus according to claim 1 or 2, **characterized in that** the container (12) has overflow channels (15) and during the first partial actuation (a) plugs (14) separating the chambers (13a, 13b) from one another can be brought into a position in the vicinity of the overflow channels (15), so that the chambers are interconnected by means of the overflow channels (15).

4. Discharge apparatus according to one of the preceding claims, **characterized in that** a connecting link guide (26) is provided for subdividing the actuating path (c) into the partial actuating paths (a, b).

5. Discharge apparatus according to claim 4, **characterized in that** the connecting link guide (26) has pressure point means (27), which have to be overcome at the start of each following partial actuation.

6. Discharge apparatus according to one of the claims 4 or 5, **characterized in that** the connecting link guide (26) for each partial actuation has a linear guide portion (42), the guide portions (42) being so mutually displaced that they are in each case limited by a stop (28) and between two partial actuations a reversing actuation differing from the partial actuation is to be performed.

7. Discharge apparatus according to claim 6, **characterized in that** at least the reversing actuations are automatically performed between two following partial actuations (b).

8. Discharge apparatus according to claim 7, **characterized in that** tension reservoirs (29) are provided and are pretensioned during the performance of a partial actuation and serve to subsequently automatically perform the reversing actuation.

9. Discharge apparatus according to one of the preceding claims, **characterized in that** the container (12) is placed in stable manner in the sleeve (34), which is movable relative to the casing (17) and is displaceable from the starting position (35) into the discharge position and in said discharge position the fluid connection is formed between the first chamber (13a) of the container and the discharge opening (18).

10. Discharge apparatus according to claim 9, **characterized in that** the bringing of the sleeve (34) from the starting position (35) into the discharge position takes place during the first partial actuation (a) and for producing the fluidic connection between the discharge opening (18) and the first chamber (13a) of the container (12) a casing-side needle (20) having a discharge channel (21) perforates a container-side plug, preferably held by a crimp closure (22).

11. Discharge apparatus according to claim 9 or 10, **characterized in that** the connecting link guide (26) is constructed between the sleeve (34) and actuating means (19) and the preferably at least one sliding block (30) is constructed on the connecting means (19) and the at least one link path (32, 33) of the connecting link guide (26) on the sleeve (34).

12. Discharge apparatus according to one of the claims 6 to 11, **characterized in that** the reversing actuation between the first partial actuation (a) and the first, following partial actuation (b) takes place by rotating the actuating means relative to the casing (12), the rotation angle being predetermined by stop edges (31) of the connecting link guide (26) and in which preferably by means of a tooth system (25a, 25b) a rotation of the sleeve (34) relative to the casing (17) is blocked.

13. Discharge apparatus according to one of the preceding claims, **characterized in that** the container (12) has a first chamber (13a) facing the discharge opening (18) of the discharge apparatus (11) and at least one further chamber (13b), and in which are provided with the plug (14) axially displaceable in the container, for separating and closing the chambers, as well as a plug (14) closing the last of the chambers (13b) and on which acts the actuating means (19).

14. Discharge apparatus according to one of the preceding claims, **characterized in that** the first chamber (13a) facing the discharge opening (18) has a volume with a gas bubble (39) and during the first partial actuation (a) by the compression of the gas bubble (39) a frictional connection can be formed between the actuating means (19) and container (12), by means of which the container (12) can be brought from the starting position (35), in which the container (12) is hermetically sealed, into the discharge position, in which a fluidic connection is produced between the discharge opening (18) and the first chamber (13a).

15. Discharge apparatus according to one of the claims 1 to 14, **characterized in that** the container (12) has two chambers (13a, 13b) and into the first chamber (13a) is preferably introduced a dry, particularly soluble media component, preferably a lyophilizate (40) and into the second chamber (13b) is introduced a carrier medium (41), preferably a liquid solvent, as a media component.

16. Discharge apparatus according to one of the preceding claims, **characterized in that** two, successive partial actuations (b), more particularly bringing about an equal discharge can be performed.

## Revendications

1. Dispositif de décharge, notamment pour des substances contenant au moins une matière active pharmaceutique, présentant un récipient (12), dans lequel est stockée la substance à décharger, sachant que le récipient (12) présente des chambres (13a, 13b) séparées, dans lesquelles se trouve respectivement un des éléments constitutifs de la substance (40, 41), qui donnent ensemble la substance à décharger, sachant que le récipient (12) est disposé dans un boîtier (17) qui présente une ouverture de décharge (18) pour la décharge de la substance, sachant qu'on prévoit un moyen d'actionnement (19) relativement mobile par rapport au boîtier (17) dont l'actionnement produit d'abord un raccord entre les chambres du récipient, sachant que la voie d'actionnement (c) du moyen d'actionnement (19) est subdivisée en une première voie d'actionnement partielle (a) et en un certain nombre de voies d'actionnement partielles (b) suivantes, sachant qu'un mélange des éléments constitutifs de la substance (40, 41) a lieu pendant la première voie d'actionnement partielle (a) pour obtenir la substance à décharger et sachant que tout actionnement partiel ultérieur du moyen d'actionnement (19) est associé à la décharge d'une dose partielle de substance bien définie, **caractérisé en ce que** le récipient (12) est disposé dans le boîtier (17) de manière déplaçable par un actionnement du moyen d'actionnement (19) à partir d'un position initiale, dans laquelle le récipient (12) est fermé de manière étanche, dans une position de décharge, dans laquelle est produit un raccord fluidique entre le récipient (12) et l'ouverture de décharge (18) en raison du déplacement, et **en ce que** l'ouverture de décharge (18) est réalisée pour une décharge nébulisée de la substance.

2. Dispositif de décharge d'après la revendication 1, **caractérisé en ce que** les chambres (13a, 13b) sont séparées au moyen de bouchons (14) disposés dans le récipient (12).

3. Dispositif de décharge d'après la revendication 1 ou 2, **caractérisé en ce que** le récipient (12) présente des conduits de by-pass (15), sachant que, pendant le premier actionnement partiel (a), les bouchons (14) séparant les chambres (13a, 13b) peuvent être portés dans une position dans le domaine des conduits de by-pass (15), de façon que les chambres sont raccordées par les conduits de by-pass (15).

4. Dispositif de décharge d'après une des revendications précédentes, **caractérisé en ce qu'**on prévoit un guidage à coulisse (26) pour la subdivision de la voie d'actionnement (c) en voies d'actionnement partielles (a, b).

5. Dispositif de décharge d'après la revendication 4, **caractérisé en ce que** le guidage à coulisse (26) présente des moyens à point de poussée (27), sachant que des moyens à point de poussée (27) sont à surmonter au début de chaque actionnement partiel ultérieur.

6. Dispositif de décharge d'après la revendication 4 ou 5, **caractérisé en ce que** le guidage à coulisse (26) présente une section de guidage (42) linéaire pour chaque actionnement partiel, sachant que les sections de guidage (42) sont décalées les unes par rapport aux autres de telle manière qu'elles sont respectivement limitées par un arrêt (28) et que, entre deux actionnements partiels, il faut effectuer respectivement un actionnement de commutation qui diffère de l'actionnement partiel.

7. Dispositif de décharge d'après la revendication 6, **caractérisé en ce qu'**au moins les actionnements de commutation entre deux actionnements partiels (b) consécutifs sont effectués automatiquement.

8. Dispositif de décharge d'après la revendication 7, **caractérisé en ce qu'**on prévoit des accumulateurs d'énergie (29), qui sont précontraints pendant un actionnement partiel et qui servent à effectuer automatiquement l'actionnement de commutation suivant.

9. Dispositif de décharge d'après une des revendications précédentes, **caractérisé en ce que** le récipient (12) est disposé en position fixe dans une douille (34), sachant que la douille (34) est relativement mobile par rapport au boîtier (17) et qu'elle est déplaçable de la position initiale (35) à la position de décharge, sachant que dans la position de décharge le raccord fluidique est établi entre la première chambre (13a) du récipient et l'ouverture de décharge (18).

10. Dispositif de décharge d'après la revendication 9, **caractérisé en ce que** le déplacement de la douille (34) de la position initiale (35) à la position de décharge à lieu pendant le premier actionnement partiel (a), sachant que pour produire le raccordement fluidique entre l'ouverture de décharge (18) et la première chambre (13a) du récipient (12), une aiguille (20) située du côté du boîtier et présentant un conduit de décharge (21) perce un bouchon situé du côté du récipient et maintenu de préférence par une fermeture à sertissage (22).

11. Dispositif de décharge d'après la revendication 9 ou 10, **caractérisé en ce que** le guidage à coulisse (26) est réalisé entre la douille (34) et le moyen d'actionnement (19) et que de préférence au moins le coulisseau (30) est réalisé sur le moyen d'actionnement (19) et qu'au moins l'une des voies de coulisse (32, 33) du guidage à coulisse (26) est réalisée sur la douille (34).

12. Dispositif de décharge d'après une des revendications de 6 à 11, **caractérisé en ce que** l'actionnement de commutation entre le premier actionnement partiel (a) et le premier actionnement partiel suivant (b) à lieu par une rotation du moyen d'actionnement par rapport au boîtier (12), sachant que l'angle de rotation est déterminé par des arêtes de butée (31) du guidage à coulisse (26) et sachant qu'une rotation de la douille (34) par rapport au boîtier (17) est empêchée de préférence par d'une denture (25a, 25b).

13. Dispositif de décharge d'après une des revendications précédentes, **caractérisé en ce que** le récipient (12) présente une première chambre (13a) orientée vers l'ouverture de décharge (18) du dispositif de décharge (11) et au moins une autre chambre (13b), qui sont dotées du bouchon (14) qui est axialement déplaçable dans le récipient et qui sépare les chambres l'une de l'autre et les referme, et d'un bouchon (14) qui referme la dernière des chambres (13b) et sur lequel agit le moyen d'actionnement (19).

14. Dispositif de décharge d'après une des revendications précédentes, **caractérisé en ce que** la première chambre (13a) orientée vers l'ouverture de décharge (18) présente un volume contenant une bulle de gaz (39), sachant que pendant le premier actionnement partiel (a) on peut produire par compression de la bulle de gaz (39) un engagement par adhérence entre le moyen d'actionnement (19) et le boîtier (12), engagement par lequel le récipient (12) peut être porté de la position initiale (35), dans laquelle le récipient (12) est fermé hermétiquement, dans la position de décharge, dans laquelle est établi un raccordement fluidique entre l'ouverture de décharge (18) et la première chambre (13a).

15. Dispositif de décharge d'après une des revendications de 1 à 14, **caractérisé en ce que** le récipient (12) présente deux chambres (13a, 13b), sachant que dans la première chambre (13a) on trouve de préférence un composant de substance sèche notamment soluble, de préférence un lyophilisat (40) et sachant que dans la deuxième chambre (13b) on trouve, en tant que composant de substance, une substance de base (41), de préférence un solvant notamment liquide.

16. Dispositif de décharge d'après une des revendications précédentes, **caractérisé en ce qu'**on peut effectuer deux actionnements partiels (b) consécutifs, qui produisent notamment une décharge de dimension identique.
